# EUROPÄISCHE PATENTSCHRIFT

(11) **EP 2 664 273 B1**
(45) Veröffentlichungstag und Bekanntmachung des Hinweises auf die Patenterteilung: **01.05.2019**
(21) Anmeldenummer: 13002610.7
(22) Anmeldetag: 17.05.2013
(51) Int. Cl.: A61B 5/00, A61B 5/0408, A61B 5/0404

(54) **Vorrichtung zur Erfassung und Speicherung von Elektrokardiogramm-Signalen**
Electrocardiogram signal recording and storage device
Dispositif destiné à l'enregistrement et au stockage de signaux d'électrocardiogramme

(30) Priorität: 17.05.2012 DE 102012009742
(43) Veröffentlichungstag der Anmeldung: 20.11.2013
(73) Patentinhaber: livetec Ingenieurbüro GmbH, 79539 Lörrach (DE)
(72) Erfinder: Schirmeier, Michael, 79650 Schopfheim (DE); Reichenbach, Klaus, 79286 Glottertal (DE)
(74) Vertreter: Geitz Truckenmüller Lucht Christ

(56) Entgegenhaltungen:
- EP-A1- 1 062 908
- WO-A1-02/22006
- WO-A1-2008/068695
- WO-A1-2009/052704
- WO-A2-2008/057884
- GB-A- 2 368 127
- US-A- 4 791 933
- US-A1- 2012 088 999

## Beschreibung

Die Erfindung geht aus von einer Vorrichtung zur Erfassung und Speicherung von Elektrokardiogramm-Signalen, welche mit an einem Gehäuse angeordneten, auf die Hautoberfläche einer Person aufklebbaren Elektroden ausgestattet ist.

Derartige Vorrichtungen dienen dazu, die Herzaktivität über einen längeren Zeitraum zu erfassen und aufzuzeichnen. Dabei wird der zeitliche Verlauf der Aktionsströme des Herzens aufgezeichnet, die durch Elektroden an der Brustwand abgeleitet werden. Die mit den Elektroden erfassten Signale werden als Elektrokardiogramm-Signale bezeichnet. Mobile Vorrichtungen werden am Körper einer Person befestigt. Sie eignen sich dazu, von einer Person über mehrere Stunden oder Tage mitgeführt zu werden. Dabei kann die Herzaktivität im normalen Alltag oder in bestimmten Belastungssituationen, wie beispielsweise bei Hochleistungssport beobachtet werden. Die erfassten Daten ermöglichen eine Diagnose von Herzerkrankungen oder liefern Informationen über die Belastbarkeit des Herzens.

Bekannte mobile Vorrichtungen zur Erfassung und Speicherung von Elektrokardiogramm-Signalen sind schwer, unhandlich und lassen sich meist schlecht über längere Zeit am Körper der zu untersuchenden Person anordnen.

Die WO 2008/057884 A2 offenbart eine am Körper zu tragende physiologische Sensorvorrichtung mit Einweg-Elektrodenmodul, mit der ein Elektrokardiogramm aufgenommen werden kann. Die Vorrichtung weist ein erstes Vorrichtungsteil mit einem Kommunikations-Berechnungs-Modul auf. Darüber hinaus weist die Vorrichtung ein zweites Vorrichtungsteil mit einer flexiblen Platine, Elektroden und Batterien auf. Dieses zweite Vorrichtungsteil wird nach Abschluss der Messung entsorgt. Das erste Vorrichtungsteil wird an das zweite Vorrichtungsteil mechanisch und elektrisch gekoppelt. Das Kommunikations-Berechnungs-Modul empfängt Signale von den Elektroden über einen Anschluss.

Die WO 02/22006 A1 offenbart ein Einwegband mit Lebenszeichenüberwachungs-Sensoren und Mehrwegelektronikmodul. Das Einwegband kann auf der Brust eines Patienten angeordnet werden und weist elektrische Leiter und mehrere Elektroden und andere Sensoren auf, um ein Elektrokardiogramm, die Atmung, die Temperatur, die Bewegung und gegebenenfalls weitere physiologische Parameter zu erfassen. Das Band wird nach der Verwendung entsorgt. Das Einwegband ist mit einer Klebeschicht ausgestattet und wird auf die Haut des Patienten aufgeklebt. An dem Einwegband ist ein zweiteiliges Gehäuse über einen Clip-Mechanismus angeordnet. In einem ersten Teil des Gehäuses sind Einwegbatterien angeordnet. Ein zweites Gehäuseteil ist mit einer wiederverwendbaren Elektronik-Schaltung ausgestattet.

Die US 2012/088999 A1 offenbart einen ambulanter Elektrokardioarafie-Monitor zur Bereitstellung einer leichten Anwendung bei Frauen und Verwendunasverfahren hierzu. Die als Monitor bezeichnete Vorrichtung weist ein Gehäuse mit einem Oberteil und einem Unterteil auf. In dem Gehäuse ist eine Leiterplatte angeordnet. Auf der Leiterplatte sind ein Prozessor, ein Speicher, eine Batterie, eine Datenschnittstelle und ein RFID-Tag angeordnet. Wird der Speicher nicht geleert oder die Batterie nicht ersetzt, ist der gesamte Monitor nach der Verwendung zu entsorgen. An der Unterseite der Leiterplatte sind Elektroden angeordnet, welche durch Öffnungen im Gehäuseunterteil nach unten überstehen. In einem zweiten Ausführungsbeispiel sind die Elektroden an sich gegenüberliegenden Enden einer dehnbaren Befestigungsplatte angeordnet, die mit einer Klebeschicht ausgestattet ist. Die Elektroden weisen Stecker auf, die in entsprechende Buchsen an der Leiterplatte passen.

Der Erfindung liegt die Aufgabe zugrunde eine Vorrichtung zur Erfassung und Speicherung von Elektrokardiogramm-Signalen zur Verfügung zu stellen, die ein geringes Gewicht und ein kleines Baumaß aufweist, schnell, einfach und zuverlässig am Körper einer Person befestigt werden kann, die handlich ist, die Person in ihrer Bewegungsfreiheit und in ihrem alltäglichen Lebensablauf nicht einschränkt und die zumindest teilweise wieder verwendet werden kann.

Diese Aufgabe wird durch eine Vorrichtung mit den Merkmalen des Anspruchs 1 gelöst. Die Vorrichtung zeichnet sich dadurch aus, dass sie zwei Vorrichtungsteile aufweist. Dabei ist ein erstes Vorrichtungsteil mit einem Elektrokardiogramm-Mess- und Speichermodul ausgestattet, welches die Erfassung von Elektrokardiogramm-Signalen steuert und die erfassten Elektrokardiogramm-Signale abspeichert. Ein zweites Vorrichtungsteil ist mit einem Energiespeicher, nämlich einer Batterie oder einem Akkumulator, ausgestattet. Jedes der beiden Vorrichtungsteile hat ein Gehäuse, an oder in dem die Komponenten angeordnet sind. An dem ersten Vorrichtungsteil sind Elektroden angeordnet, welche auf die Hautoberfläche einer Person aufklebbar sind. Dabei kann es sich um Einweg-Elektroden handeln, die an das erste Vorrichtungsteil elektrisch leitend und mechanisch gekoppelt werden und nach Beendigung der Datenaufzeichnung entsorgt werden. Die Elektroden sind elektrisch leitend mit dem Elektrokardiogramm-Mess- und -Speichermodul verbindbar. Hierzu ist das Vorrichtungsteil, an welches die Elektroden koppelbar sind, mit einer elektrisch leitenden Verbindung ausgestattet, welche die Position, an welche die Elektroden mechanisch koppeln, mit dem Elektrokardiogramm-Mess- und Speichermodul verbindet.

Bevor die Vorrichtung bei einer Person am ihrem Körper angeordnet wird, werden die beiden Vorrichtungsteile miteinander verbunden. Die Vorrichtung ist mit einer mechanischen Kopplungseinrichtung ausgestattet, mit der das zweite Vorrichtungsteil an das erste Vorrichtungsteil lösbar mechanisch koppelbar ist. Die Kopplungseinrichtung ist derart ausgestaltet, dass sich das erste und das zweite Vorrichtungsteil nicht unerwünscht voneinander lösen können. Hierzu ist das erste und/ oder das zweite Vorrichtungsteil beispielsweise mit einer Schnapp-, Klemm-, Spann-, Schraub- oder Rasteinrichtung ausgestattet. Nach der Beendigung der Messung kann das zweite Vorrichtungsteil von dem ersten Vorrichtungsteil gelöst werden, ohne dass die beiden Vorrichtungsteile dabei beschädigt werden.

Das erste Vorrichtungsteil ist mit einem Elektrokardiogramm-Mess- und - Speichermodul ausgestattet. Dieses zeichnet die durch die Herztätigkeit ausgelösten Spannungsdifferenzen oder Ströme an den Elektroden auf. Hierzu wird das Elektrokardiogramm-Mess- und Speichermodul über eine elektrisch leitende Verbindung mit den Elektroden verbunden. Die Energie hierfür liefert der Energiespeicher, welcher im zweiten Vorrichtungsteil angeordnet ist. Das Elektrokardiogramm-Mess- und Speichermodul enthält vorteilhafterweise einen Verstärker, einen Ananlog-Digital-Wandler, einen Controller, einen Prozessor und einen Datenspeicher. Es ist beispielsweise ausgelegt, um vier EKG-Signale der Elektroden mit einer Abtastrate von bis zu 500 S/s über die Dauer von mindestens acht Tagen aufzuzeichnen. Möglich sind auch bis zu 1000 S/s.

Das erste Vorrichtungsteil kann mit einem in dem ersten Gehäuse angeordneten elektrischen Schaltkreis ausgestattet sein. An diesen ist das Elektrokardiogramm-Mess- und -Speichermodul elektrisch leitend angeschlossen. Darüber hinaus kann der Schaltkreis auch Teil des Elektrokardiogramm-Mess- und - Speichermoduls sein.

Der in dem zweiten Vorrichtungsteil angeordnete Energiespeicher ist mit dem Elektrokardiogramm-Mess- und -Speichermodul elektrisch leitend verbunden, wenn das erste und das zweite Vorrichtungsteil elektrisch leitend miteinander verbunden sind. Der Energiespeicher liefert die von dem Elektrokardiogramm-Mess- und -Speichermodul für die Aufzeichnung eines Elektrokardiogramms benötigte Energie.

Die Vorrichtung ist mit einer Einrichtung zum lösbaren elektrisch leitenden Verbinden des ersten Vorrichtungsteils mit dem zweiten Vorrichtungsteil ausgestattet. Diese Einrichtung sorgt dafür, dass das Elektrokardiogramm-Mess- und -Speichermoduls mit dem Energiespeicher elektrisch leitend verbunden werden. Die Vorrichtung ist somit mit einer Einrichtung zum lösbaren elektrisch leitenden Verbinden des Elektrokardiogramm-Mess- und - Speichermoduls mit dem Energiespeicher ausgestattet. Die elektrisch leitende Verbindung zwischen dem ersten und zweiten Vorrichtungsteil kommt bevorzugt bei der mechanischen Kopplung des ersten und zweiten Vorrichtungsteils zustande, ohne dass hierzu weitere Handgriffe notwendig sind. Die Einrichtung zum lösbaren elektrisch leitenden Verbinden kann Teil der mechanischen Kopplungseinrichtung sein. Dank der elektrisch leitenden Verbindung wird das Elektrokardiogramm-Mess- und -Speichermodul an den Energiespeicher des zweiten Vorrichtungsteils angeschlossen.

Da die elektrisch leitende Verbindung zwischen dem Energiespeicher und dem Elektrokardiogramm-Mess- und -Speichermodul erst zustande kommt, wenn das erste und das zweite Vorrichtungsteil über die mechanische Kopplungseinrichtung mechanisch miteinander verbunden werden, wird verhindert, dass sich der Energiespeicher bereits entlädt, bevor eine Messung durchgeführt wird.

Die Vorrichtung ist mit einer Schnittstelle an dem ersten Vorrichtungsteil ausgestattet, über welche Daten zwischen dem Elektrokardiogramm-Mess- und -Speichermodul und einem externen Lesegerät ausgetauscht werden können. Das Lesegerät kann auch ein Schreib-Lesegerät sein. Insbesondere kann es sich hierbei um einen Computer handeln. Der Datenaustausch kann dabei mit einem Kabel oder kabellos, beispielsweise über Funk, erfolgen.

Die Elektroden sind an dem ersten Vorrichtungsteil angeordnet. Sie sind elektrisch leitend mit dem Elektrokardiogramm-Mess- und -Speichermodul verbindbar. Die Elektroden an können direkt mit dem Elektrokardiogramm-Mess- und -Speichermodul elektrisch leitend verbunden sein.

Zum Erfassen eines Elektrokardiogramms wird das zweite Vorrichtungsteil an dem ersten Vorrichtungsteil mechanisch befestigt und elektrisch leitend mit diesem verbunden. Dann wird die Vorrichtung am Körper einer Person befestigt, in dem die Elektroden auf die Hautoberfläche im Bereich der Brust oder der Gliedmaßen aufgeklebt werden. Hierzu weisen die Elektroden ebene Oberflächen auf, die großflächig ausgebildet sind. Der Klebekontakt mit der Hautoberfläche kommt daher großflächig zustande, so dass für eine gute Haftung der Vorrichtung am Körper der Person gesorgt ist. Die Vorrichtung ist klein, handlich und leicht, so dass der Klebekontakt zur Befestigung ausreicht. Die Vorrichtung beeinträchtigt die Person in ihrer Beweglichkeit nicht. Die Spannungsdifferenzen zwischen den Elektroden oder die zwischen den Elektroden fließenden Ströme, welche durch die Herzaktivität ausgelöst werden, werden als Elektrokardiogramm-Signale durch das Elektrokardiogramm-Mess- und Speichermodul aufgezeichnet. Diese Aufzeichnung kann über mehrere Tage dauern. Ist die Aufzeichnung beendet, so werden die in dem Speicher des Elektrokardiogramm-Mess- und Speichermoduls abgelegten Daten über die Schnittstelle mit einem externen Lesegerät ausgetauscht.

Hierbei handelt es sich bevorzugt um einen Computer. Dort können die erfassten Daten weiter ausgewertet und ausgegeben werden. Vor oder nach dem Auslesen der Daten können die beiden Vorrichtungsteile voneinander getrennt werden.

Das erste Vorrichtungsteil kann wieder verwendet werden. Hierzu werden die in dem Elektrokardiogramm-Mess- und -Speichermodul gespeicherten Daten einer Person gelöscht. Damit enthält das Elektrokardiogramm-Mess- und Speichermodul keine individuellen Daten mehr. Es steht für eine erneute Aufzeichnung eines Elektrokardiogramms zur Verfügung.

Das zweite Vorrichtungsteil kann so ausgestaltet sein, dass es nach Beendigung einer Aufzeichnung eines Elektrokardiogramms entsorgt werden muss. Dies gilt insbesondere dann, wenn der Energiespeicher nicht aufladbar ist. Kann der Energiespeicher ersetzt oder wieder aufgeladen werden, so kann das zweite Vorrichtungsteil ebenfalls wieder verwendbar sein.

Die Elektroden können so ausgestaltet sein, dass sie in der Regel nach dem Gebrauch entsorgt werden.

Nach einer vorteilhaften Ausgestaltung der Erfindung ist das Elektrokardiogramm-Mess- und -Speichermodul als physikalisches und logisches Laufwerk ausgestaltet, das sich beim Auslesen der Daten durch ein externes Schreib-Lesegerät einfach ansteuern und aufrufen lässt. Es verhält sich beim Auslesen der Daten durch das Schreib-/ Lesegerät wie ein physikalisches und logisches Laufwerk, ähnlich oder identisch zu einem SD-Card-Reader oder eine PC-Festplatte, so dass die Daten mit einer hohen Datenübertragungsrate ausgelesen werden können. Es weist eine Logik auf, die das Auslesen der in dem Speicher des Elektrokardiogramm-Mess- und -Speichermoduls abgespeicherten Daten mit hoher Datenrate unterstützt.

Nach einer weiteren vorteilhaften Ausgestaltung der Erfindung umfasst das Elektrokardiogramm-Mess- und -Speichermodul einen Datenspeicher, einen Prozessor, einen A/D-Wandler und einen Taktgeber oder eine Uhr. Über den Taktgeber oder die Uhr wird die Zeit erfasst, so dass eine zeitliche Zuordnung der aufgenommenen Daten möglich ist.

Nach einer weiteren vorteilhaften Ausgestaltung der Erfindung ist das Elektrokardiogramm-Mess- und -Speichermodul mit einem Verstärker ausgestattet. Dieser verstärkt die Spannungsdifferenzen zwischen den Elektroden oder die zwischen den Elektroden fließenden Ströme, welche die Signale darstellen.

Nach einer weiteren vorteilhaften Ausgestaltung der Erfindung ist an den Elektroden eine Klebeschicht angeordnet, über welche die Elektroden auf der Hautoberfläche einer Person befestigt werden.

Vorteilhafterweise wird zur Überprüfung des Kontaktes zwischen den Elektroden und der Hautoberfläche der zu untersuchenden Person eine Impedanzmessung durchgeführt. Diese Messung kann durchgeführt werden, wenn die Elektroden am Körper einer Person befestigt werden, wenn eine Elektrode gewechselt wird oder wenn es Probleme bei der Aufnahme von Elektrokardiogramm-Signale gibt. Auf diese Weise kann ein schlechter Elektrodenkontakt, welcher zu schlechten Messergebnissen führt, erkannt, nachgewiesen und gegebenenfalls angezeigt werden, sofern eine Anzeigeeinrichtung vorhanden ist.

Nach einer weiteren vorteilhaften Ausgestaltung sind die Elektroden lösbar mit dem ersten Vorrichtungsteil verbunden. In diesem Fall können bekannte Elektroden verwendet und an der Vorrichtung angeordnet werden. Die Elektroden können mit bekannten EKG-Druckknöpfen ausgestattet sein, über welche ein mechanischer und ein elektrisch leitender Kontakt zu dem ersten oder zweiten Vorrichtungsteil hergestellt wird. Hierzu weist das Gehäuse des ersten Vorrichtungsteils für jede Elektrode eine Vertiefung auf. In der Vertiefung sind eine oder mehrere Federn angeordnet. Die Elektroden sind mit einem in die Vertiefung passenden Kopf ausgestattet. Alternativ dazu kann das erste Vorrichtungsteil mit jeweils einem Kopf für eine Elektrode ausgestattet sein. In diesem Fall weist jede Elektrode eine Vertiefung zur Aufnahme eines Kopfes auf. Diese Vertiefung ist mit einer oder mehreren Federn ausgestattet. Beim Einführen des Kopfes in die Vertiefung werden die Federn ausgelenkt. Ist der Kopf vollständig in der Vertiefung aufgenommen, gehen die Federn wieder in ihre Ausgangsstellung zurück. Der Kopf wird damit durch die Federkraft in der Vertiefung gehalten. Über die Federn kann eine elektrisch leitende Verbindung zwischen der Elektrode und dem ersten Vorrichtungsteil hergestellt werden.

Nach einer weiteren vorteilhaften Ausgestaltung der Erfindung sind die Elektroden unmittelbar an dem ersten Vorrichtungsteil unter Vermeidung zusätzlicher elektrischer Leiter angeordnet. Sie befinden sich damit bei einer an einer Person angeordneten Vorrichtung unterhalb der Vorrichtung. Dadurch wird ein kleines Baumaß der Vorrichtung erreicht.

Nach einer weiteren vorteilhaften Ausgestaltung der Erfindung sind an dem ersten Vorrichtungsteil Kopplungseinrichtungen angeordnet, an welchen die Elektroden mechanisch befestigbar sind. Die Kopplungseinrichtungen können auch als Befestigungseinrichtungen bezeichnet werden. Darüber hinaus ist das erste oder zweite Vorrichtungsteil mit einer Leiterplatte ausgestattet. Die Kopplungseinrichtungen sind in die Leiterplatte integriert. Auf diese Weise kommt über die mechanischen Kopplungseinrichtungen auch zugleich eine elektrisch leitende Verbindung mit der Leiterplatte und den auf der Leiterplatte angeordneten elektrischen Komponenten zustande. Hierbei kann es sich um den Energiespeicher oder das Elektrokardiogram-Mess- und - Speichermodul handeln. Sind mit Druckknöpfen aufgestattete Elektroden vorgesehen, so kann die Aufnahme für den Druckknopf in das erste Vorrichtungsteil integriert sein.

Nach einer weiteren vorteilhaften Ausgestaltung der Erfindung ist das erste oder zweite Vorrichtungsteil mit einer oder mehreren Lichtquellen zur Statusanzeige ausgestattet. Hierbei kann es sich beispielsweise um Licht emittierende Dioden LEDs handeln. Es können mehrere LEDs unterschiedlicher Farbe vorgesehen sein um unterschiedliche Zustände anzuzeigen. Folgende Zustände können beispielsweise angezeigt werden:
- Anzeige des Vorrichtungszustands wie Inbetriebnahme oder Datenaufnahme,
- Ergebnisse eines von dem Elektrokardiogramm-Mess- und - Speichermoduls nach Herstellen einer elektrisch leitenden Verbindung zwischen dem Energiespeicher und dem Elektrokardiogramm-Mess- und Speichermodul durchgeführten Selbsttests,
- Zustände und Fehler während des Betriebs der Vorrichtung wie beispielsweise codierte Ausgabe der Elektrokardiogramm-Signale über die LEDs auf Anfrage via Pulsweitenmodulation,
- Zustand des Energiespeichers,
- Zustand des Speichers des Elektrokardiogramm-Mess- und Speichermoduls,
- Qualität der elektrisch leitenden Verbindung zwischen den Elektroden und dem Körper der zu untersuchenden Person, Überprüfung durch Impedanzmessung,
- Abspeicherung der Elektrokardiogramm-Signale im Speicher des Elektrokardiogramm-Mess- und Speichermoduls,
- Zugriff auf den Speicher des Elektrokardiogramm-Mess- und Speichermoduls,
- Status des Beschleunigungssensors,
- Datenaustausch zwischen dem Elektrokardiogramm-Mess- und Speichermodul und einem externen Schreib- Lesegerät über die Schnittstelle.

Nach einer weiteren vorteilhaften Ausgestaltung der Erfindung ist die Einrichtung zum lösbaren elektrisch leitenden Verbinden zwischen dem ersten und dem zweiten Vorrichtungsteil als Federkontakt ausgebildet. Darüber hinaus sind andere Kontakte möglich.

Nach einer weiteren vorteilhaften Ausgestaltung der Erfindung ist die Schnittstelle eine drahtgebundene Schnittstelle. Mit dieser können hohe Datenübertragungsraten realisiert werden. Es sind beispielsweise bekannte Schnittstellen mit Kabel wie USB-, Ethernet- oder UART-Schnittstellen möglich.

Alternativ dazu kann die Schnittstelle auch eine kabellose Schnittstelle, beispielsweise Bluetooth oder Infrarot, sein. Eine kabellose Schnittstelle bietet den Vorteil, dass ein Datenaustausch mit einem externen Lesegerät auch während der Aufzeichnung eines Elektrokardiogramms stattfinden kann, da zum Datenaustausch nicht der zweite Vorrichtungsteil vom ersten Vorrichtungsteils entfernt werden muss.

Nach einer weiteren vorteilhaften Ausgestaltung der Erfindung ist das erste Vorrichtungsteil mit einer Vertiefung ausgestattet, in welcher das zweite Vorrichtungsteil angeordnet ist. Im Bereich der Vertiefung umschließt das erste Vorrichtungsteil das zweite Vorrichtungsteil an mehreren Seiten. Alternativ dazu kann das zweite Vorrichtungsteil mit einer Vertiefung ausgestattet sein, in welcher das erste Vorrichtungsteil angeordnet ist. Die Vertiefung befindet sich dabei an der Außenseite des ersten oder zweiten Gehäuses. Nach einer weiteren vorteilhaften Ausgestaltung der Erfindung ist die Vertiefung in dem ersten oder zweiten Vorrichtungsteil an der den Elektroden abgewandten Seite angeordnet ist. Somit wird das eine Vorrichtungsteil beim Verbinden mit dem anderen Vorrichtungsteil von oben in die Vertiefung eingesetzt. Das erste oder das zweite Vorrichtungsteil ist damit auch während der Aufzeichnung eines Elektrokardiogramms von außen sichtbar. So können Lichtquellen zur Statusanzeige stets eingesehen werden. Das Status der Vorrichtung kann somit zu jedem beliebigen Zeitpunkt visuell überprüft werden.

Nach einer weiteren vorteilhaften Ausgestaltung der Erfindung ist die Vorrichtung mit einem Bewegungssensor und/ oder einem Beschleunigungssensor ausgestattet. Dieser erfasst Körperbewegungen der zu untersuchenden Person. Diese Bewegungen werden ebenfalls aufgezeichnet und können bei einer Auswertung in Korrelation gesetzt werden zu dem Elektrokardiogramm. Es können Bewegungsartefakte detektiert werden. Mittels des Beschleunigungssensors kann die Aufzeichnung der Elektrokardiogramm-Signalen gestartet werden. Hierzu kann beispielsweise manuell zwei oder drei Mal auf das Gehäuse des ersten oder zweiten Vorrichtungsteils geklopft werden. Der Beschleunigungssensor erfasst diese Erschütterungen. Stimmen sie mit einem vorgegebenen Muster an Erschütterungen überein, so wird die Aufzeichnung der Elektrokardiogramm-Signale ausgelöst. Es können unterschiedliche charakteristische Erschütterungen in dem Elektrokardiogramm-Mess- und - Speichermodul abgespeichert sein. Jeder dieser charakteristischen Erschütterungen kann eine spezielle Aktion zugeordnet sein, wie beispielsweise die Durchführung einer speziellen zusätzlichen Messung oder die Anzeige der aufgenommenen Elektrokardiogramm-Signale mittels der an der Vorrichtung angeordneten Lichtquellen.

Nach einer weiteren vorteilhaften Ausgestaltung der Erfindung ist die Vorrichtung mit einem Temperatursensor ausgestattet ist. Dieser erfasst die Körpertemperatur einer Person. Die Körpertemperatur wird aufgezeichnet und kann bei der Auswertung in Korrelation gesetzt werden zu dem Elektrokardiogramm. Darüber hinaus können weitere physikalische Daten der Person erfasst werden, zu der ein Elektrokardiogramm aufgezeichnet wird. Hierzu kann die Vorrichtung gegebenenfalls mit weiteren Sensoren ausgestattet sein.

Nach einer weiteren vorteilhaften Ausgestaltung der Erfindung ist die Vorrichtung wasserfest. Sie kann somit von einer Person auch während des Duschens getragen werden.

Nach einer weiteren vorteilhaften Ausgestaltung der Erfindung sind alle Elektroden in ein auf die Haut einer Person aufklebbares, zusammenhängendes Pflaster integriert. Dies erleichtert das Anordnen der Elektroden auf der Haut einer Person in den richtigen, durch die Vorrichtung vorgegebenen Abständen. Darüber hinaus weist ein zusammenhängendes Pflaster eine große Klebefläche auf, so dass die Elektroden zuverlässig an der Person haften. Das Pflaster kann aus einem geschäumten Material, wie beispielsweise PU-Schaum, oder aus einem Gewebe bestehen. In bevorzugter Weise weist das Pflaster eine viereckige oder dreieckige Form auf, wobei die Elektroden im wesentlichen an oder nahe der Ecken an dem Pflaster angeordnet sind. Darüber hinaus kann das Pflaster auch rund sein.

Weitere Vorteile und vorteilhafte Ausgestaltungen der Erfindung sind der nachfolgenden Beschreibung, der Zeichnung und den Ansprüchen entnehmbar.

### Zeichnung

Ein Ausführungsbeispiel der Erfindung ist in der Zeichnung dargestellt. Es zeigen:
- Figur 1: nicht erfindungsgemäße Vorrichtung zur Erfassung und Speicherung von Elektrokardiogramm-Signalen in einer Ansicht von oben,
- Figur 2: Vorrichtung gemäß Figur 1 in einer Ansicht von der Seite,
- Figur 3: zweites Vorrichtungsteil der Vorrichtung gemäß Figur 1 in einer Ansicht von oben,
- Figur 4: zweites Vorrichtungsteil gemäß Figur 3 in Schnittdarstellung,
- Figur 5: erstes Vorrichtungsteil der Vorrichtung gemäß Figur 1 in einer Ansicht von oben,
- Figur 6: erstes Vorrichtungsteil gemäß Figur 5 in Schnittdarstellung,
- Figur 7: erstes Vorrichtungsteil gemäß Figur 5 in einer Ansicht von unten,
- Figur 8: erstes und zweites Vorrichtungsteil gemäß Figuren 4 und 6 unmittelbar vor dem Zusammenfügen,
- Figur 9: erstes und zweites Vorrichtungsteil gemäß Figuren 4 und 6 im zusammengebauten Zustand,
- Figur 10: erstes Vorrichtungsteil gemäß Figur 6 unmittelbar vor dem Einsetzen in ein Schreib-Lesegerät,
- Figur 11: erstes Vorrichtungsteil gemäß Figur 6 eingesetzt in ein Schreib-Lesegerät gemäß Figur 10,
- Figur 12: erfindungsgemäßes Ausführungsbeispiel einer Vorrichtung zur Erfassung und Speicherung von Elektrokardiogramm-Signalen in einer Ansicht von der Seite,
- Figur 13: Elektroden der Vorrichtung gemäß Figur 12 in einer Ansicht von unten.

### Beschreibung des Ausführungsbeispiels

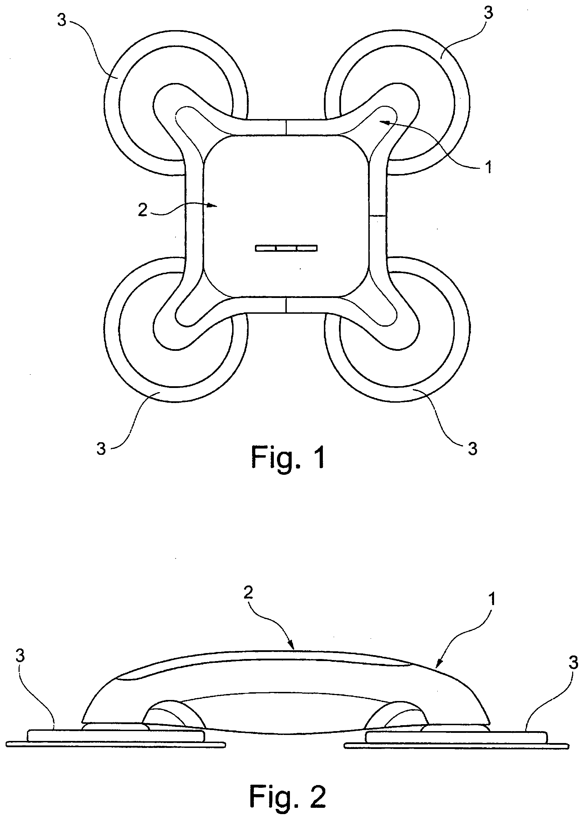

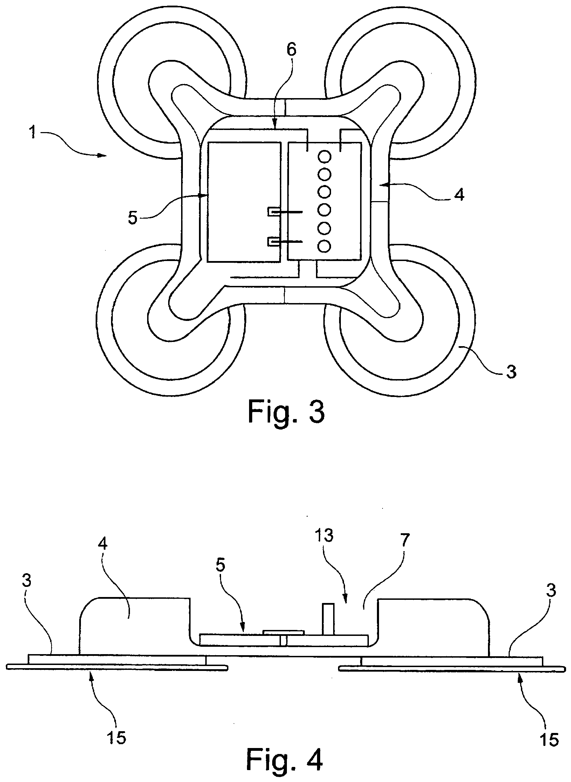

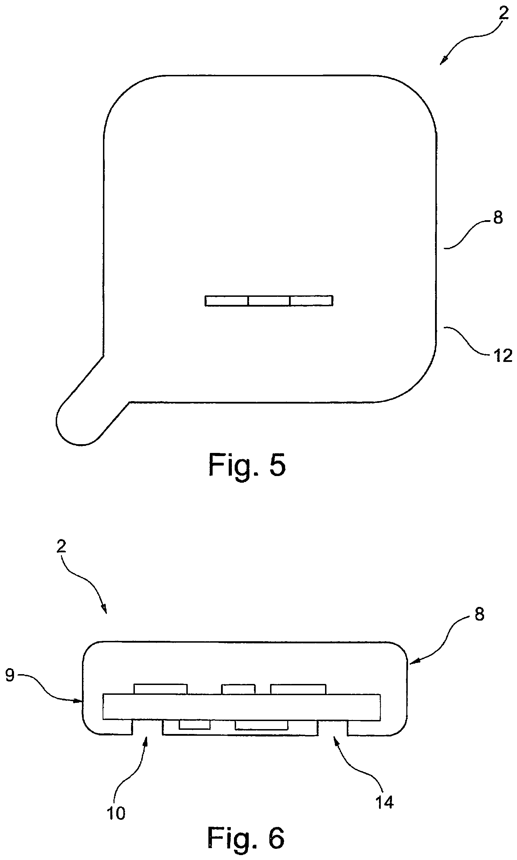

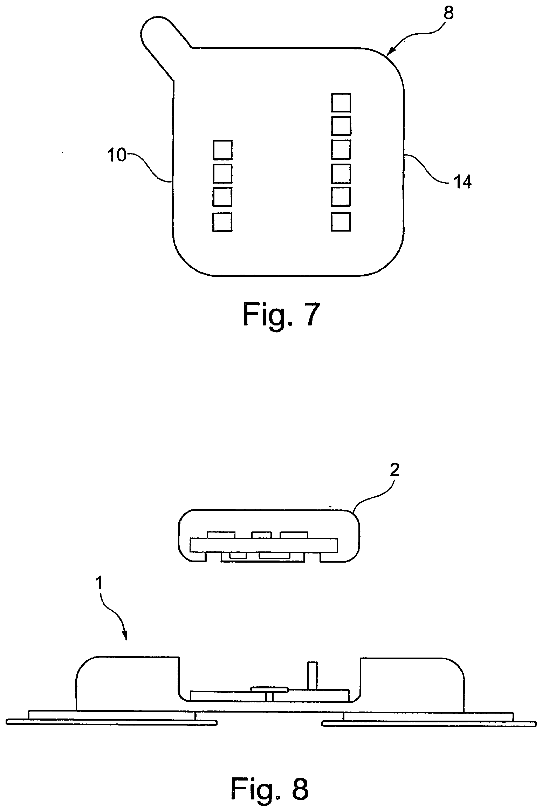

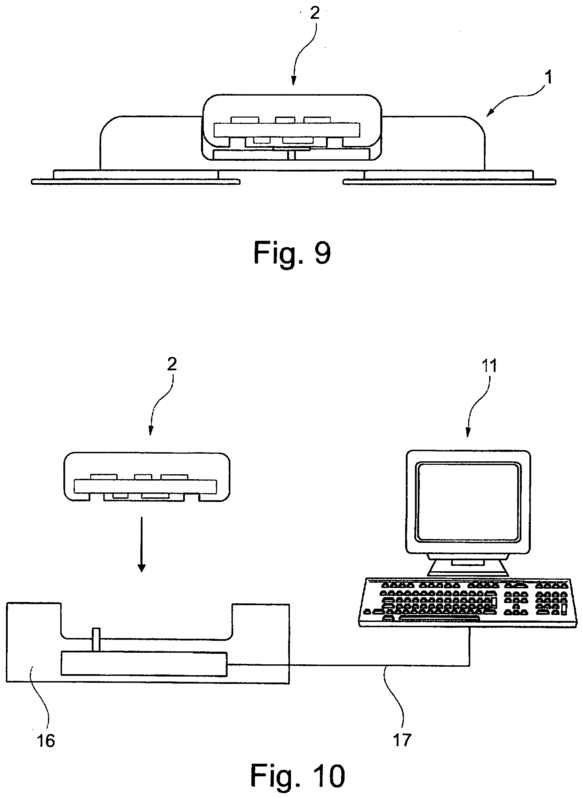

In den Figuren 1 bis 11 ist ein nicht erfindungsgemäßes Ausführungsbeispiel einer mobilen Vorrichtung zur Erfassung und Speicherung von Elektrokardiogramm-Signalen in verschiedenen Ansichten dargestellt. Die Vorrichtung weist ein erstes Vorrichtungsteil 2 und ein zweites Vorrichtungsteil 1 auf. Das zweite Vorrichtungsteil 1 ist mit vier Elektroden 3 und einem zweiten Gehäuse 4 ausgestattet, in welchem ein Energiespeicher 5 und eine elektrische Schaltung 6 angeordnet sind. Ferner weist das zweite Gehäuse 4 des zweiten Vorrichtungsteils 1 an der den Elektroden 3 abgewandten Seite eine Vertiefung 7 auf, welche in den Figuren 4 und 8 besonders gut erkennbar ist. In dieser Vertiefung 7 ist das erste Vorrichtungsteil 2 im zusammengefügten Zustand der Vorrichtung angeordnet. Dies ist besonders gut in Figur 9 erkennbar. Das erste Vorrichtungsteil 2 weist ein erstes Gehäuse 8 auf, in dem ein Elektrokardiogramm-Mess- und -Speichermodul 9 und eine Schnittstelle 10 angeordnet sind. Mit dem Elektrokardiogramm-Mess- und -Speichermodul 9 wird ein Elektrokardiogramm einer Person aufgezeichnet und abgespeichert. Die Schnittstelle 10 dient dazu, Daten zwischen dem Elektrokardiogramm-Mess- und -Speichermodul 9 und einem externen Schreib-Lesegerät 11 auszutauschen. Das erste Vorrichtungsteil 2 ist ferner mit Lichtquellen 12 in Form von LEDs ausgestattet, welche den Status der Vorrichtung im zusammengebauten Zustand anzeigen.

Die Vorrichtung weist eine mechanische Kopplungseinrichtung auf, mit der das erste Vorrichtungsteil 2 an das zweite Vorrichtungsteil 1 lösbar mechanisch gekoppelt werden kann. In der gekoppelten Stellung ist das erste Vorrichtungsteil 2 in der Vertiefung 7 des zweiten Vorrichtungsteils 1 gehalten. Die mechanische Kopplungseinrichtung weist mehrere in der Zeichnung nicht dargestellte Rastnasen an dem ersten Vorrichtungsteil 2 auf, welche in ebenfalls nicht dargestellte Vertiefungen des zweiten Vorrichtungsteils 1 eingreifen. Die mechanische Kopplung kann gelöst werden, indem die Rastnasen aus den Vertiefungen herausgeführt werden.

Die Einrichtung zum lösbaren elektrisch leitenden Verbinden des ersten und zweiten Vorrichtungsteils weist mehrere Stifte 13 an dem zweiten Vorrichtungsteil 1 auf, die in entsprechende Vertiefungen 14 an dem ersten Vorrichtungsteil 2 eingreifen. Dabei rasten die Stifte 13 in der mechanisch gekoppelten Stellung derart in den Vertiefungen 14 ein, dass das erste Vorrichtungsteil 2 an dem zweiten Vorrichtungsteil 1 sicher gehalten ist und sich nicht unerwünscht von diesem lösen kann. Die Stifte 13 sind als Federkontakt ausgestaltet. Ferner weisen die Vertiefungen 14 Kontakte auf. Sind die Stifte 13 in den Vertiefungen 14 angeordnet, so kommt eine elektrisch leitende Verbindung zwischen den Federkontakten des ersten Vorrichtungsteils und den Kontakten des zweiten Vorrichtungsteils zustande. Die Federkontakte der Stifte 13 sind wiederum elektrisch leitend mit der Schaltung 6 verbunden. Die Kontakte der Vertiefungen 14 sind elektrisch leitend mit dem Elektrokardiogramm-Mess- und - Speichermodul 9 verbunden.

Der Energiespeicher 5 versorgt alle elektrischen Komponenten der Vorrichtung mit Spannung und/ oder Strom. Dies gilt insbesondere für das Elektrokardiogramm-Mess- und -Speichermodul 9 und die Lichtquellen 12.

Die Elektroden 3, das zweite Gehäuse 4, das erste Gehäuse 8 und die mechanische Kopplung des zweiten Vorrichtungsteils 2 an das erste Vorrichtungsteil 1 sind wasserfest ausgestaltet.

Die Elektroden 3 weisen an der der Vertiefung 7 des zweiten Gehäuses 4 abgewandten Seite jeweils eine kreisrunde Kontaktfläche 15 auf, die mit einer Klebeschicht ausgestattet ist. Diese Klebeschicht haftet an der Hautoberfläche einer Person. Über diese Klebeschicht wird die Vorrichtung mit dem Körper einer Person verbunden. Im Bereich dieser Klebeschicht sind elektrische Kontakte vorgesehen, über die eine elektrisch leitende Verbindung zwischen der Haut der Person und der Vorrichtung hergestellt wird.

Figuren 1, 2 und 9 zeigen die Vorrichtung in der mechanisch und elektrisch gekoppelten Stellung des ersten und zweiten Vorrichtungsteils 1, 2. In dieser Stellung kann die Vorrichtung auf die Haut einer Person aufgeklebt werden. Hierzu werden die Elektroden derart ausgerichtet, dass die elektrischen Erregungen des Herzens mit Hilfe der Elektroden erfasst werden können. Die Lichtquellen 12 zeigen dabei an, ob die Vorrichtung ein Elektrokardiogramm aufzeichnet.

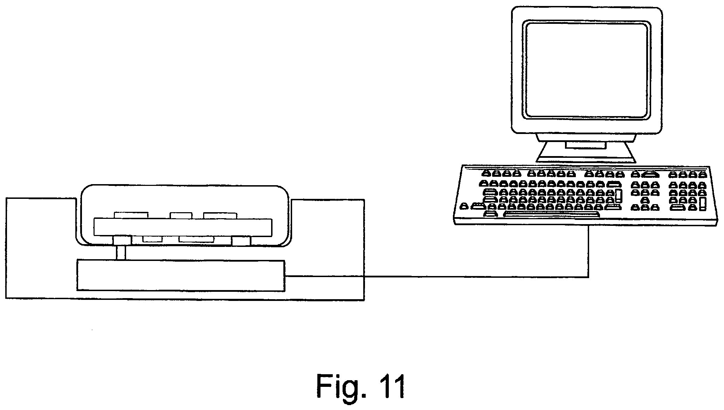

Nach Beendigung des Messvorgangs wird die Vorrichtung von der Haut der Person abgenommen. Anschließend wird das erste Vorrichtungsteil 2 von dem zweiten Vorrichtungsteil mechanisch und elektrisch entkoppelt und von diesem entfernt. Dies entspricht der Darstellung gemäß Figur 8. Das zweite Vorrichtungsteil kann anschließend entsorgt werden. Das erste Vorrichtungsteil wird, wie in den Figuren 10 und 11 dargestellt, mit einem Schreib-Lesegerät 11 verbunden. Der Datenaustausch erfolgt über die Schnittstelle 10 an dem ersten Vorrichtungsteil 2. Bei dem Schreib-Lesegerät 11 handelt es sich um einen Computer, der mit einem Adapter 16 ausgestattet ist. In den Adapter 16 wird das erste Vorrichtungsteil 2 eingelegt, so dass zwischen der Schnittstelle 10 und dem Adapter 16 eine Verbindung zum Datenaustausch aufgebaut werden kann. Diese Daten werden über ein Kabel 17 an das Schreib-Lesegerät 11 weitergegeben. Über den Adapter 16 und die Schnittstelle 10 können auch Daten von dem Schreib-Lesegerät in das Elektrokardiogramm-Mess- und - Speichermodul 9 des ersten Vorrichtungsteils 2 eingelesen werden. Dies gilt beispielsweise für die Daten der Person, deren Elektrokardiogramm aufgezeichnet werden soll.

Nachdem die Messung beendet und die während der Messung mit der Vorrichtung aufgezeichneten Daten auf das Schreib-Lesegerät 11 übertragen wurden, kann das erste Vorrichtungsteil 2 erneut für eine Messung verwendet werden. Hierzu wird es bevorzugt mit einem neuen zweiten Vorrichtungsteil 1 verbunden. Bevor es für eine erneute Messung zur Verfügung steht, können die in dem Elektrokardiogramm-Mess- und -Speichermodul 9 abgespeicherten Daten betreffend das aufgezeichnete Elektrokardiogramm einer ersten Person gelöscht werden.

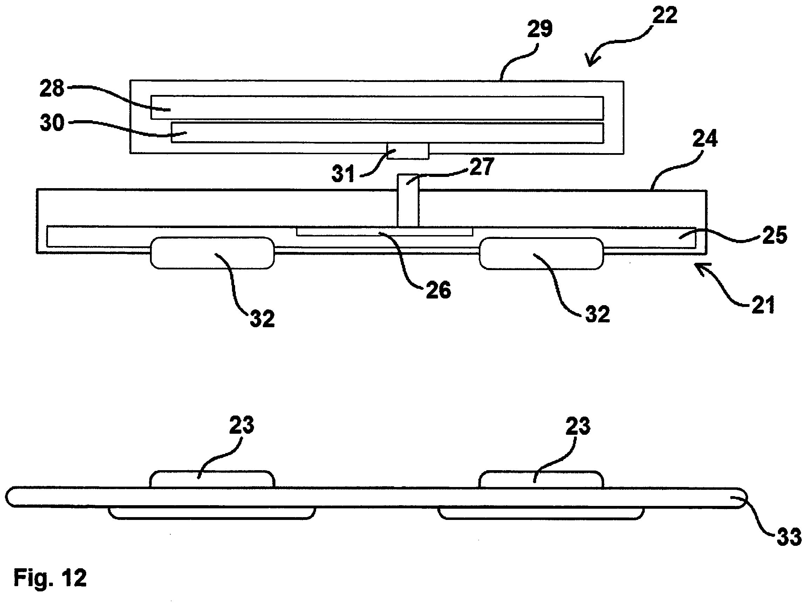

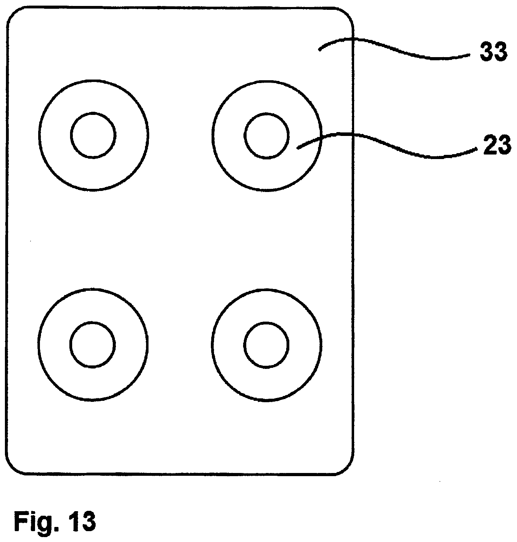

In den Figuren 12 und 13 ist ein erfindungsgemäßes Ausführungsbeispiel einer mobilen Vorrichtung zur Erfassung und Speicherung von Elektrokardiogramm-Signalen dargestellt. Die Vorrichtung weist ein erstes Vorrichtungsteil 21, ein zweites Vorrichtungsteil 22 und Elektroden 23 auf. Figur 12 zeigt die Vorrichtung in einer Ansicht von der Seite, wobei das erste Vorrichtungsteil 21, das zweite Vorrichtungsteil 22 und die Elektroden 23 voneinander getrennt sind. Im Unterschied zu dem nicht erfindungsgemäßen Ausführungsbeispiel werden die Elektroden an dem ersten Vorrichtungsteil 21 angeordnet. Das erste Vorrichtungsteil 21 ist mit einem ersten Gehäuse 24 ausgestattet, in welchem eine Platine 25 angeordnet ist. Auf dieser Platine 25 befindet sich ein Elektrokardiogramm-Mess- und - Speichermodul 26. Das Elektrokardiogramm-Mess- und -Speichermodul 26 ist in eine elektrische Schaltung auf der Leiterplatte bzw. Platine 25 eingebettet. Das erste Vorrichtungsteil ist ferner mit einer Schnittstelle 27 ausgestattet. Über diese Schnittstelle 27 ist das Elektrokardiogramm-Mess- und -Speichermodul 26 des ersten Vorrichtungsteils 21 mit einem Energiespeicher 28 verbindbar. Der Energiespeicher 28 ist in einem Gehäuse des zweiten Vorrichtungsteils 22 angeordnet. Das Gehäuse des zweiten Vorrichtungsteils 22 wird zur Unterscheidung von dem ersten Gehäuse 24 des ersten Vorrichtungsteils 21 als zweites Gehäuse 29 bezeichnet. Das zweite Vorrichtungsteil 22 ist ebenfalls mit einer Platine 30 ausgestattet. Der Energiespeicher 28 ist als Batterie ausgebildet. Diese ist auf die Platine 30 aufgelötet. Das zweite Vorrichtungsteil 22 ist mit einer Schnittstelle 31 ausgestattet. Die Schnittstelle 31 ist mit der Platine 30 elektrisch leitend verbunden.

Bei den Schnittstellen 27, 31 des ersten und zweiten Vorrichtungsteils 21, 22 handelt es sich um elektrische Kontakte. Diese können als Pad, Stift oder Feder ausgebildet sein.

Das erste Vorrichtungsteil 21 weist an seiner dem zweiten Vorrichtungsteil 22 abgewandten Seite Kopplungseinrichtungen 32 auf. Diese können auch als Befestigungseinrichtungen bezeichnet werden. Über diese Kopplungseinrichtungen 32 werden die Elektroden 23 mechanisch und elektrisch leitend an das erste Vorrichtungsteil 21 gekoppelt. Die mechanische Kopplung kommt nach Art eines bekannten Druckknopfes zustande. Da die Kopplungseinrichtungen 32 und das Gegenstück der Elektroden 23 aus Metall bestehen, kommt bei der mechanischen Kopplung auch eine elektrisch leitende Verbindung zustande. Diese Art der Kopplung nach Art eines Druckknopfes hat den Vorteil, dass die betreffenden Teile einfach zu koppeln und entkoppeln sind. Die Kopplungseinrichtungen 32 sind elektrisch leitend mit der Platine 25 verbunden. Sie können beispielsweise auf die Platine aufgelötet sein.

In Figur 13 sind die Elektroden 23 in einer Ansicht von unten dargestellt. Die dem Betrachter zugewandte Seite wird auf die Haut einer Person aufgesetzt. Alle vier Elektroden sind an einem viereckigen, zusammenhängenden Pflaster 33 angeordnet. Das Pflaster 33 ist rechteckig. Die Elektroden 23 sind in einem Quadrat angeordnet.

Zur Aufzeichnung eines Elektrokardiogramms werden das erste und zweite Vorrichtungsteil 21, 22 mechanisch miteinander verbunden und über die Schnittstellen 27, 31 elektrisch leitend miteinander verbunden. Darüber hinaus werden die Elektroden 23 an die Kopplungseinrichtung 32 des ersten Vorrichtungsteils 21 gekoppelt. Das Pflaster 33 wird auf der Haut einer Person positioniert und aufgeklebt.

Die Funktionalität der einzelnen Komponenten entspricht darüber hinaus der Funktionalität der Komponenten des nicht erfindungsgemäßen Ausführungsbeispiels gemäß Figuren 1 bis 11. Das erfindungsgemäße Ausführungsbeispiel kann genau wie das nicht erfindungsgemäße Ausführungsbeispiel mit weiteren Komponenten wie Lichtquellen und Sensoren ausgestattet sein. Das Auslesen eines aufgezeichneten Elektrokardiogramms erfolgt entsprechend zum nicht erfindungsgemäßen Ausführungsbeispiel.

Sämtliche Merkmale der Erfindung können sowohl einzeln als auch in beliebiger Kombination miteinander erfindungswesentlich sein.

### Bezugszahlen

- 1: zweites Vorrichtungsteil
- 2: erstes Vorrichtungsteil
- 3: Elektroden
- 4: zweites Gehäuse
- 5: Energiespeicher
- 6: elektrische Schaltung
- 7: Vertiefung
- 8: erstes Gehäuse
- 9: Elektrokardiogramm-Mess- und -Speichermodul
- 10: Schnittstelle
- 11: externes Schreib-Lesegerät
- 12: Lichtquelle
- 13: Stift der mechanischen Kopplungseinrichtung
- 14: Vertiefung der mechanischen Kopplungseinrichtung
- 15: Kontaktfläche der Elektrode
- 16: Adapter
- 17: Kabel
- 18:
- 19:
- 20:
- 21: erstes Vorrichtungsteil
- 22: zweites Vorrichtungsteil
- 23: Elektroden
- 24: Erstes Gehäuse
- 25: Platine
- 26: Elektrokardiogramm-Mess- und -Speichermodul
- 27: Schnittstelle
- 28: Energiespeicher
- 29: zweites Gehäuse
- 30: Platine
- 31: Schnittstelle
- 32: Kopplungsvorrichtung mit Elektrischer Kontakt
- 33: Pflaster

## Patentansprüche

1. Vorrichtung zur Erfassung und Speicherung von Elektrokardiogramm-Signalen
mit einem ersten Vorrichtungsteil (21) mit einem ersten Gehäuse (24), mit einem in dem ersten Gehäuse (24) angeordneten Elektrokardiogramm-Mess- und - Speichermodul (26), welches die Erfassung von Elektrokardiogramm-Signalen steuert und die erfassten Elektrokardiogramm-Signale in einem Datenspeicher speichert,
mit einer Schnittstelle (27) an dem ersten Vorrichtungsteil (21), über welche Daten zwischen dem Elektrokardiogramm-Mess- und - Speichermodul (26) und einem externen Lesegerät austauschbar sind, mit einem zweiten Vorrichtungsteil (22) mit einem zweiten Gehäuse (29), mit einem an oder in dem zweiten Gehäuse (29) des zweiten Vorrichtungsteils (22) angeordneten Energiespeicher (28), welcher als Batterie oder Akkumulator ausgebildet ist,
mit einer mechanischen Kopplungseinrichtung, mit der das zweite Vorrichtungsteil (22) an das erste Vorrichtungsteil (21) lösbar mechanisch koppelbar ist,
mit einer Einrichtung zum lösbaren elektrisch leitenden Verbinden des Elektrokardiogramm-Mess- und - Speichermoduls (26) mit dem Energiespeicher (28),
mit Elektroden (23), welche an das erste Vorrichtungsteil (21) mechanisch koppelbar sind und welche auf die Hautoberfläche einer Person aufklebbar sind,
wobei die Elektroden (23) elektrisch leitend mit dem Elektrokardiogramm-Mess- und -Speichermodul (26) verbindbar sind,
wobei das erste Vorrichtungsteil (21) an seiner dem zweiten Vorrichtungsteil (22) abgewandten Seite Kopplungseinrichtungen (32) aufweist, über welche die Elektroden (23) mechanisch und elektrisch leitend an das erste Vorrichtungsteil (21) gekoppelt sind.

2. Vorrichtung nach Anspruch 1, **dadurch gekennzeichnet, dass** das Elektrokardiogramm-Mess- und -Speichermodul (26) einen Datenspeicher, einen Prozessor, einen A/D-Wandler und einen Taktgeber oder eine Uhr umfasst.

3. Vorrichtung nach Anspruch 1 oder 2, **dadurch gekennzeichnet, dass** das Elektrokardiogramm-Mess- und -Speichermodul (26) mit einem Verstärker ausgestattet ist.

4. Vorrichtung nach Anspruch 1, 2 oder 3, **dadurch gekennzeichnet, dass** an den Elektroden (23) eine Klebeschicht angeordnet ist, über welche die Elektroden (23) auf der Hautoberfläche einer Person befestigbar sind.

5. Vorrichtung nach einem der vorhergehenden Ansprüche, **dadurch gekennzeichnet, dass** die Elektroden (23) unmittelbar an dem ersten Vorrichtungsteil (21, 22) angeordnet sind.

6. Vorrichtung nach einem der vorhergehenden Ansprüche, **dadurch gekennzeichnet, dass** das erste oder zweite Vorrichtungsteil (21, 22) mit einer oder mehreren Lichtquellen (12) zur Statusanzeige ausgestattet ist.

7. Vorrichtung nach einem der vorhergehenden Ansprüche, **dadurch gekennzeichnet, dass** das erste Vorrichtungsteil (21) mit einer elektrischen Schaltung ausgestattet ist, welches Teil des Elektrokardiogramm-Mess- und Speichermoduls (26) ist, oder welches zusätzlich zu dem Elektrokardiogramm-Mess- und -Speichermodul (26) in dem ersten Vorrichtungsteil (21) angeordnet und elektrisch leitend mit dem Elektrokardiogramm-Mess- und Speichermodul (26) verbunden ist, und dass der Energiespeicher (28) und die Elektroden (23) an die elektrische Schaltung elektrisch leitend gekoppelt sind.

8. Vorrichtung nach einem der vorhergehenden Ansprüche, **dadurch gekennzeichnet, dass** sie mit einer Einrichtung zum lösbaren elektrisch leitenden Verbinden des ersten und zweiten Vorrichtungsteils (21, 22) ausgestattet ist, und dass die Einrichtung als Federkontakt ausgebildet ist.

9. Vorrichtung nach einem der vorhergehenden Ansprüche, **dadurch gekennzeichnet, dass** die Schnittstelle (27, 31) eine drahtgebundene Schnittstelle ist.

10. Vorrichtung nach einem der vorhergehenden Ansprüche, **dadurch gekennzeichnet, dass** sie mit einem Beschleunigungssensor ausgestattet ist.

11. Vorrichtung nach einem der vorhergehenden Ansprüche, **dadurch gekennzeichnet, dass** sie mit einem Temperatursensor ausgestattet ist.

12. Vorrichtung nach einem der vorhergehenden Ansprüchen, **dadurch gekennzeichnet, dass** sie wasserfest ist.

13. Vorrichtung nach einem der vorhergehenden Ansprüchen, **dadurch gekennzeichnet, dass** erste Gehäuse (24) und/ oder das zweite Gehäuse (29) flexibel und formstabil sind.

14. Vorrichtung nach einem der vorhergehenden Ansprüche, **dadurch gekennzeichnet, dass** alle Elektroden (23) in ein auf die Haut einer Person aufklebbares, zusammenhängendes Pflaster (33) integriert sind.

15. Vorrichtung nach Anspruch 14, **dadurch gekennzeichnet, dass** das Pflaster (33) viereckig oder dreieckig ist.

## Claims

1. Electrocardiogram signal recording and storage device
with a first device part (21) with a first housing (24),
with an electrocardiogram measuring and storage module (26) arranged in the first housing (24) which controls the recording of electrocardiogram signals and stores the recorded electrocardiogram signals in a data storage,
with an interface (27) on the first device part (21) over which data can be exchanged between the electrocardiogram measuring and storage module (26) and an external reader,
with a second device part (22) with a second housing (29),
with an energy storage (28) arranged on or in the second housing (29) of the second device part (22) which takes the form of a battery or rechargeable battery,
with a mechanical coupling device with which the second device part (22) can be detachably mechanically coupled to the first device part (21), with a device for the detachable conductive connection of the electrocardiogram measuring and storage module (26) to the energy storage (28),
with electrodes (23) which can be mechanically coupled to the first device part (21) and which can be adhered to a person's skin surface, whereby the electrodes (23) can be conductively connected to the electrocardiogram measuring and storage module (26),
whereby the first device part (21) has coupling devices (32) on the side facing the second device part (22) over which the electrodes (23) are mechanically and conductively coupled to the first device part (21).

2. Device according to claim 1, **characterised in that** the electrocardiogram measuring and storage module (26) comprises a data storage, a processor, an A/D converter and a pulse generator or a clock.

3. Device according to claim 1 or 2, **characterised in that** the electrocardiogram measuring and storage module (26) is equipped with an amplifier.

4. Device according to claim 1, 2 or 3, **characterised in that** the electrodes (23) have an adhesive layer with which the electrodes (23) can be affixed to a person's skin surface.

5. Device according to one of the previous claims, **characterised in that** the electrodes (23) are arranged directly on the first device part (21, 22).

6. Device according to one of the previous claims, **characterised in that** the first or second device part (21, 22) is equipped with one or more light sources (12) for the status display.

7. Device according to one of the previous claims, **characterised in that** the first device part (21) is equipped with an electric circuit which is part of the electrocardiogram measuring and storage module (26) or which is arranged in the first device part (21) in addition to the electrocardiogram measuring and storage module (26) and is conductively connected to the electrocardiogram measuring and storage module (26), and that the energy storage (28) and the electrodes (23) are conductively coupled to the electric circuit.

8. Device according to one of the previous claims, **characterised in that** it is equipped with a device for the detachable conductive connection of the first and second device part (21, 22), and that the device takes the form of a spring contact.

9. Device according to one of the previous claims, **characterised in that** the interface (27, 31) is a wired interface.

10. Device according to one of the previous claims, **characterised in that** it is equipped with an acceleration sensor.

11. Device according to one of the previous claims, **characterised in that** it is equipped with a temperature sensor.

12. Device according to one of the previous claims, **characterised in that** it is waterproof.

13. Device according to one of the previous claims, **characterised in that** the first housing (24) and/or the second housing (29) is/are flexible and dimensionally stable.

14. Device according to one of the previous claims, **characterised in that** all electrodes (23) are integrated into an interconnected patch (33) which can be adhered to a person's skin.

15. Device according to claim 14, **characterised in that** the patch (33) is square or triangular.

## Revendications

1. Dispositif destiné à l'enregistrement et au stockage de signaux d'électrocardiogramme
avec une première partie de dispositif (21) dotée d'un premier boîtier (24), avec un module de mesure et de stockage des données d'électrocardiogramme (26) disposé dans le premier boîtier (24), qui commande l'enregistrement des signaux d'électrocardiogramme et stocke les signaux d'électrocardiogramme enregistrés dans une mémoire de données,
avec une interface (27) sur la première partie de dispositif (21), qui permet l'échange de données entre le module de mesure et de stockage des données d'électrocardiogramme (26) et un lecteur externe,
avec une seconde partie de dispositif (22) dotée d'un second boîtier (29), avec une réserve d'énergie (28) disposée sur ou dans le second boîtier (29) de la seconde partie de dispositif (22) et conçue en tant que pile ou accumulateur,
avec un élément de couplage mécanique permettant de coupler mécaniquement et de manière amovible la seconde partie de dispositif (22) à la première partie de dispositif (21),
avec un élément servant à établir une liaison électrique conductrice amovible entre le module de mesure et de stockage des données d'électrocardiogramme (26) et la réserve d'énergie (28),
avec des électrodes (23) pouvant être couplées mécaniquement à la première partie de dispositif (21) et être collées sur la surface de la peau d'une personne,
lesdites électrodes (23) pouvant être reliées par une liaison électrique conductrice au module de mesure et de stockage des données d'électrocardiogramme (26),
ladite première partie de dispositif (21) présentant, du côté opposé à la seconde partie de dispositif (22), des éléments de couplage (32) servant à coupler les électrodes (23) à la première partie de dispositif (21) par une liaison mécanique et électrique conductrice.

2. Dispositif selon la revendication 1, **caractérisé en ce que** le module de mesure et de stockage des données d'électrocardiogramme (26) comprend une mémoire de données, un processeur, un convertisseur analogique-numérique et un générateur d'horloge ou une horloge.

3. Dispositif selon la revendication 1 ou 2, **caractérisé en ce que** le module de mesure et de stockage des données d'électrocardiogramme (26) est doté d'un amplificateur.

4. Dispositif selon la revendication 1, 2 ou 3, **caractérisé en ce que** les électrodes (23) sont dotées d'une couche adhésive permettant de fixer les électrodes (23) sur la surface de la peau d'une personne.

5. Dispositif selon l'une quelconque des revendications précédentes, **caractérisé en ce que** les électrodes (23) sont disposées directement sur la première partie de dispositif (21, 22).

6. Dispositif selon l'une quelconque des revendications précédentes, **caractérisé en ce que** la première ou la seconde partie de dispositif (21, 22) est dotée d'une ou de plusieurs sources lumineuses (12) servant à indiquer l'état.

7. Dispositif selon l'une quelconque des revendications précédentes, **caractérisé en ce que** la première partie de dispositif (21) est dotée d'un circuit électrique qui fait partie du module de mesure et de stockage des données d'électrocardiogramme (26) ou qui est disposé dans la première partie de dispositif (21) en complément au module de mesure et de stockage des données d'électrocardiogramme (26) et relié par une liaison électrique conductrice au module de mesure et de stockage des données d'électrocardiogramme (26), et **en ce que** la réserve d'énergie (28) et les électrodes (23) sont couplées au circuit électrique par une liaison électrique conductrice.

8. Dispositif selon l'une quelconque des revendications précédentes, **caractérisé en ce qu'**il est doté d'un élément servant à établir une liaison électrique conductrice amovible entre la première et la seconde partie de dispositif (21, 22), et **en ce que** cet élément est un contact à ressort.

9. Dispositif selon l'une quelconque des revendications précédentes, **caractérisé en ce que** l'interface (27, 31) est une interface filaire.

10. Dispositif selon l'une quelconque des revendications précédentes, **caractérisé en ce qu'**il est doté d'un capteur d'accélération.

11. Dispositif selon l'une quelconque des revendications précédentes, **caractérisé en ce qu'**il est doté d'un capteur de température.

12. Dispositif selon l'une quelconque des revendications précédentes, **caractérisé en ce qu'**il est étanche à l'eau.

13. Dispositif selon l'une quelconque des revendications précédentes, **caractérisé en ce que** le premier boîtier (24) et/ou le second boîtier (29) est souple et indéformable.

14. Dispositif selon l'une quelconque des revendications précédentes, **caractérisé en ce que** toutes les électrodes (23) sont intégrées dans un patch (33) d'un seul tenant pouvant être collé sur la peau d'une personne.

15. Dispositif selon la revendication 14, **caractérisé en ce que** le patch (33) est de forme carrée ou triangulaire.
